(19) **Europäisches Patentamt**

**European Patent Office**

**Office européen des brevets**

(11) **EP 1 274 361 B1**

(12) **EUROPÄISCHE PATENTSCHRIFT**

(45) Veröffentlichungstag und Bekanntmachung des Hinweises auf die Patenterteilung:
**20.08.2003 Patentblatt 2003/34**

(51) Int Cl.⁷: **A61B 19/00**, A61B 17/02, F16M 11/14

(21) Anmeldenummer: **01944995.8**

(22) Anmeldetag: **05.04.2001**

(86) Internationale Anmeldenummer:
**PCT/EP01/03883**

(87) Internationale Veröffentlichungsnummer:
**WO 01/078617 (25.10.2001 Gazette 2001/43)**

(54) **FLEXIBLE SPANNVORRICHTUNG, INSBESONDERE FÜR MEDIZINISCHE ZWECKE**

FLEXIBLE TENSIONING DEVICE, ESPECIALLY FOR MEDICAL PURPOSES

DISPOSITIF DE SERRAGE SOUPLE, NOTAMMENT POUR DES USAGES MEDICAUX

(84) Benannte Vertragsstaaten:
**DE FR GB IT**

(30) Priorität: **19.04.2000 DE 10019321**

(43) Veröffentlichungstag der Anmeldung:
**15.01.2003 Patentblatt 2003/03**

(73) Patentinhaber: **Karl Storz GmbH & Co. KG 78532 Tuttlingen (DE)**

(72) Erfinder:
• **SALVERMOSER, Markus 78532 Tuttlingen (DE)**
• **BACHER, Uwe 78532 Tuttlingen (DE)**

(74) Vertreter: **Hofmeister, Frank Horst Kleiststrasse 7 40878 Ratingen (DE)**

(56) Entgegenhaltungen:
**CH-A- 437 461          DE-U- 29 720 250
US-A- 897 349          US-A- 3 278 207
US-A- 3 858 578**

EP 1 274 361 B1

**Beschreibung**

Die Erfindung betrifft eine flexible Spannvorrichtung, insbesondere für medizinische Zwecke, mit einem aus mehreren gegeneinander verschwenkbaren Gliederelementen bestehenden Arm, wobei der Arm in der jeweiligen Verschwenkstellung der Gliederelemete über ein Spannelement arretierbar ist.

Derartige flexible Spannvorrichtungen sind insbesondere aus der Medizin bekannt, wo sie dazu dienen, ein medizinisches Werkzeug, wie beispielsweise einen Spatel zum Öffnen von Wundrändern eines Operationsgebiets, festzuhalten. Diese bekannten Spannvorrichtungen bestehen im wesentlichen aus Kugeln und Rohrstücken, die im Inneren von einer Litze durchzogen sind. Zum Arretieren des Arms in der jeweiligen Position wird die Litze gespannt, wodurch ein Reibschluß zwischen den Kugeln und den Rohrstücken entsteht, der den Arm in der eingestellten Stellung versteift. Nachteilig bei diesen bekannten flexiblen Spannvorrichtungen ist, daß aufgrund der im Inneren verlaufenden Litze die Hohlräume sowie die Litze fast unmöglich zu reinigen sind.

Eine gattungsgemäße Spannvorrichtung ist aus der US 897,349 bekannt. Bei dieser bekannten Spannvorrichtung sind die einzelnen Gliederelemente in Längsrichtung dreiteilig aufgebaut und weisen an einem Ende eine Gelenkkugel und am gegenüberliegenden Ende eine Gelenkpfanne zur Aufnahme einer Gelenkkugel auf. Die beiden die Gelenkpfanne bildenden Längsteile eines jeden Gliederelements sind über jeweils zwei die Längsteile durchdringende Bolzen gegeneinander festlegbar, wobei über die Bolzen gleichzeitig ein die Gelenkkugel tragender Schaft innerhalb der beiden Längsteile eines Gliederelements fixierbar ist. Jedes Gliederelement dieser bekannten Spannvorrichtung besteht somit aus drei Bauteilen, nämlich den zwei Längsteilen und dem die Gelenkkugel tragenen Schaft. Für die Montage jedes Gliederelements bedarf es einer sehr exakten Fertigung mit engem Toleranzbereich, da es zum Zusammenbau der Gliederelemente zwingend notwendig ist, daß die Bohrungen in den beiden Längsteilen und in dem Schaft genau zueinander ausgerichtet sind, damit diese Bauteile über die Bolzen miteinander verbunden werden können. Somit sind die einzelnen Gliederelemente dieser Spannvorrichtung einerseits fertigungstechnisch nur mit großem Aufwand exakt herstellbar und bedarf es andererseits einer aufwendigen handwerklichen Tätigkeit, um die einzelnen Bauteile jedes Gliederelements zusammenzufügen, wodurch die Herstellungskosten für eine solche bekannte Spannvorrichtung sehr hoch sind.

US-3858578 offenbart eine flexible Spannvorrichtung gemäß dem Oberbegriff des ersten Anspruchs.

Ausgehend von diesem Stand der Technik liegt der Erfindung die **Aufgabe** zugrunde, eine flexible Spannvorrichtung der eingangs genannten Art zu schaffen, die einerseits vielseitig verstellbar und arretierbar ist und darüber hinaus einfach aufgebaut ist, um eine einfache Montage und eine vollständige Reinigung zu ermöglichen.

Die **Lösung** dieser Aufgabenstellung ist erfindungsgemäß dadurch gekennzeichnet, daß jedes Gliederelement in Längsrichtung zweiteilig aufgebaut ist, wobei die einzelnen Längsteile zum Arretieren gegeneinander verstellbar sind.

Durch die erfindungsgemäße Verwendung der Gelenkkugel-Gelenkpfanne-Verbindungen zum Verbinden der einzelnen Gliederelemente ist ein Verstellen des erfindungsgemäßen spannbaren Arms mit möglichst vielen Freiheitsgraden erreichbar. Ferner ist es möglich, den Arm in jeder dieser Positionen auf einfache Art und Weise dadurch zu arretieren, daß die Form der Gelenkkugeln durch Verstellen der Längsteile eines jeden Gliederelements zueinander verformt wird. Der einfache zweiteilige Aufbau der Gliederelemente ohne innenliegende Bauteile und anderweitige Hohlräume erleichtert weiterhin die Montage und Reinigbarkeit der erfindungsgemäßen flexiblen Spannvorrichtung.

Gemäß einer bevorzugten Ausführungsform der Erfindung wird vorgeschlagen, daß die Längsteile der in Längsrichtung zweiteilig aufgebauten Gliederelemente in der Seitenansicht in etwa Z-förmig so ausgebildet sind, daß bei einem Längsteil der obere Teil der Gelenkkugel mit dem unterer Teil der Gelenkpfanne und bei dem anderen Längsteil desselben Gliederelements der untere Teil der Gelenkkugel mit dem oberen Teil der Gelenkpfanne verbunden ist.

Zum gegenseitigen Verstellen der Längsteile eines Gliederelements weisen die Gliederelemente eine beiden Längsteilen gemeinsame Achse auf. Diese gemeinsame Schwenkachse stellt sicher, daß einerseits das Gelenkkugelteil und das Gelenkpfannenteil eines jeden Längsteils im gleichen Maß verstellt werden und andererseits auch die Verstellung der Längsteile zueinander gleichmäßig erfolgt.

Die Achse zum Verschwenken der Längsteile eines jeden Gliederelements ist in der Mitte des Gliederelements ausgebildet und in der Ebene des Längsschnitts durch Gelenkkugel und Gelenkpfanne so ausgebildet, daß die Längsteile so gegeneinander verschwenkbar sind, daß die Gelenkkugel und die Gelenkpfanne einen Öffnungswinkel $\alpha$ zwischen den Längsteilen ausbildend spreizbar sind. Durch das Aufspreizen der Gelenkpfanne wird so automatisch die Gelenkkugel um das gleiche Maß aufgespreizt, was zu einem Verklemmen in der Gelenkpfanne führt, in der die Gelenkkugel gelagert ist. Dieses gegenseitige Verklemmen führt zum arretierenden Festlegen des flexiblen Arms in der jeweiligen Verschwenkstellung.

Weiterhin wird mit der Erfindung vorgeschlagen, daß die Öffnungswinkel ausgehend vom proximalen Gliederelement hin zum distalen Gliederelement von Gliederelement zu Gliederelement mit

$$\alpha_{prox} > \alpha_n > \alpha_{n+1} > \cdots > \alpha_{dist} \cong 0$$

abnehmen.

Um die Gliederelemente beim gegenseitigen Verstellen einerseits zu führen und andererseits den Verschwenkwinkel zu begrenzen, ist rechtwinklig zur Längsteilung der Gelenkkugel durch den Kugeläquator eines jeden Gliederelements eine Durchgangsbohrung ausgebildet, in die pro Längsteil der Gelenkkugel ein nach außen über die äußere Oberfläche der Gelenkkugel herausragender Stift einsetzbar, der im mit einem anderen Gliederelement verbundenen Zustand in einer in der Gelenkpfanne ausgebildeten Nut geführt wird.

Zum Verstellen und Spannen des flexiblen Arms ist an das das proximale Ende des Arms bildende Gliederelement eine ein Spannelement bildende Handhabe anschließbar, über die die proximale Gelenkpfanne aufspreizbar ist.

Schließlich wird mit der Erfindung vorgeschlagen, daß am das distale Ende des Arms bildende Gliederelement ein Werkzeug festlegbar ist.

Weitere Merkmale und Vorteile der Erfindung ergeben sich aus der nachfolgenden Beschreibung der zugehörigen Zeichnung, in der ein Ausführungsbeispiel einer erfindungsgemäße ausgebildeten flexiblen Spannvorrichtung beispielhaft schematisch dargestellt ist. In der Zeichnung zeigt:

Fig. 1      eine perspektivische Seitenansicht einer erfindungsgemäßen flexiblen Spannvorrichtung mit proximaler Handhabe und distalem Werkzeug;

Fig. 2      eine vergrößerte und teilweise geschnittene ausschnittweise Detailansicht der Darstellung gemäß Fig. 1;

Fig. 3a      eine teilweise geschnittene und vergrößerte Seitenansicht eines Gliederelements und

Fig. 3b      eine um 90° gedrehte ungeschnittene Seitenansicht des Gliederelements gemäß Fig. 3a.

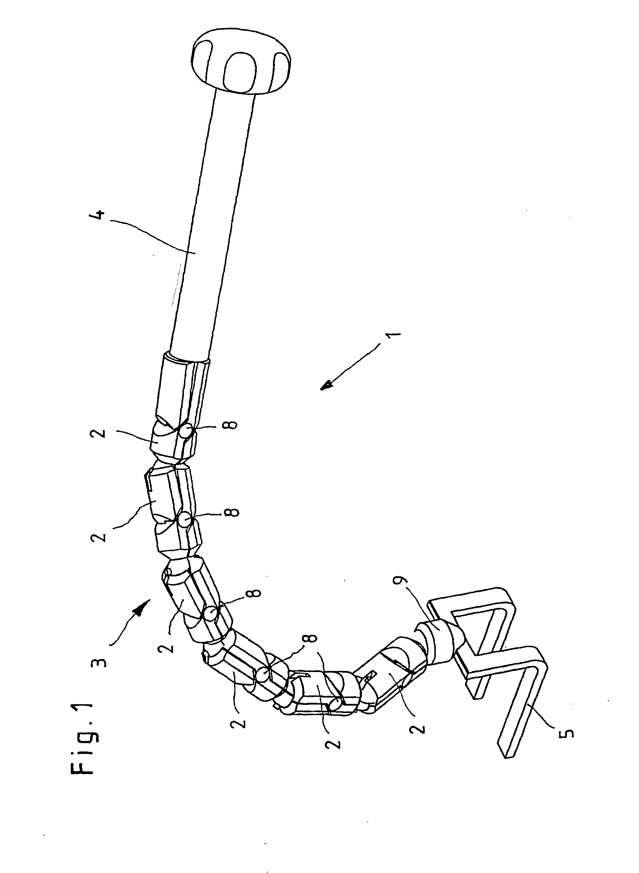

Die Darstellung in Fig. 1 zeigt eine perspektivische Ansicht einer flexiblen Spannvorrichtung 1, wie sie insbesondere in der Medizin verwendet wird, um beispielsweise ein Werkzeug ortsfest und sicher zu halten. Die dargestellte Spannvorrichtung 1 besteht im wesentlichen aus einem aus mehreren gegeneinander verschwenkbaren Gliederelementen 2 aufgebauten Arm 3, an dessen proximalem Ende eine Handhabe 4 und an dessen distalem Ende ein Werkzeug 5 festgelegt sind.

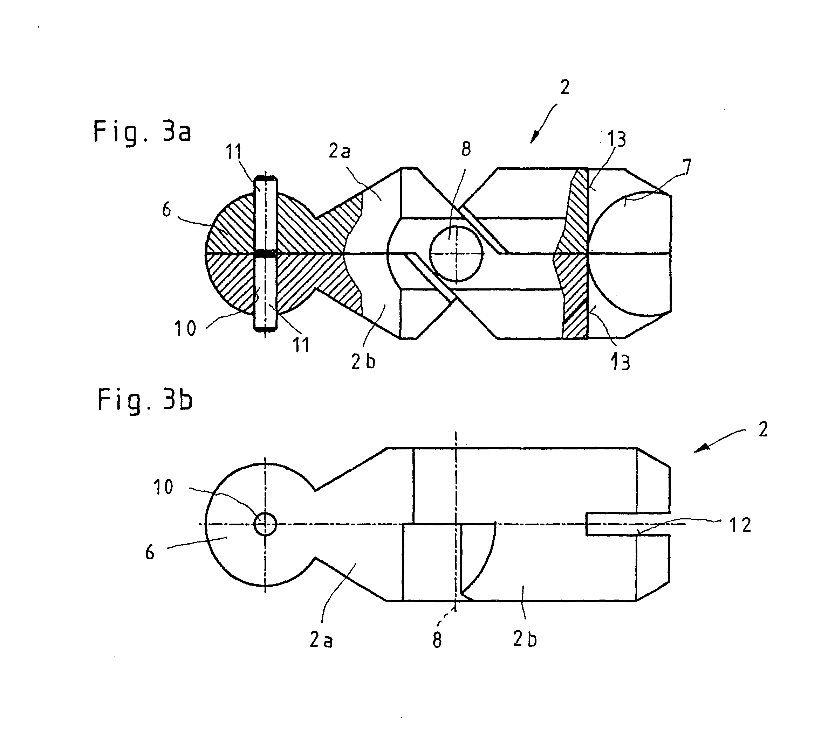

Wie insbesondere aus Fig. 3a und 3b ersichtlich, weist jedes Gliederelement 2 an einem Ende eine Gelenkkugel 6 und am gegenüberliegenden Ende eine Gelenkpfanne 7 zur Aufnahme einer Gelenkkugel 6 auf.

Über diese Kugelgelenkverbindungen verfügt der flexible Arm 3 über möglichst viele Freiheitsgrade, so daß der Arm 3, wie aus Fig. 1 ersichtlich, nahezu beliebig verdreht und verschwenkt werden kann.

Um den verschwenkten Arm 3 in jeder beliebigen verschwenkten Stellung arretieren zu können und somit ein weiteres Verstellen der Gliederelemente 2 gegeneinander zu unterbinden, ist jedes Gliederelement 2 in Längsrichtung geteilt zweiteilig aufgebaut, wie dies den Darstellungen gemäß Fig. 3a und 3b zu entnehmen ist. Die beiden Längsteile 2a und 2b jedes Gliederelements 2 sind bei dem dargestellten Ausführungsbeispiel in der Seitenansicht Z-förmig ausgebildet, wobei die Zweiteilung so ausgeführt ist, daß beim Längsteil 2a der obere Teil der Gelenkkugel 6 mit dem unteren teil der Gelenkpfanne 7 und beim Längsteil 2b desselben Gliederelements 2 der untere Teil der Gelenkkugel 6 mit dem oberen Teil der Gelenkpfanne 7 verbunden ist.

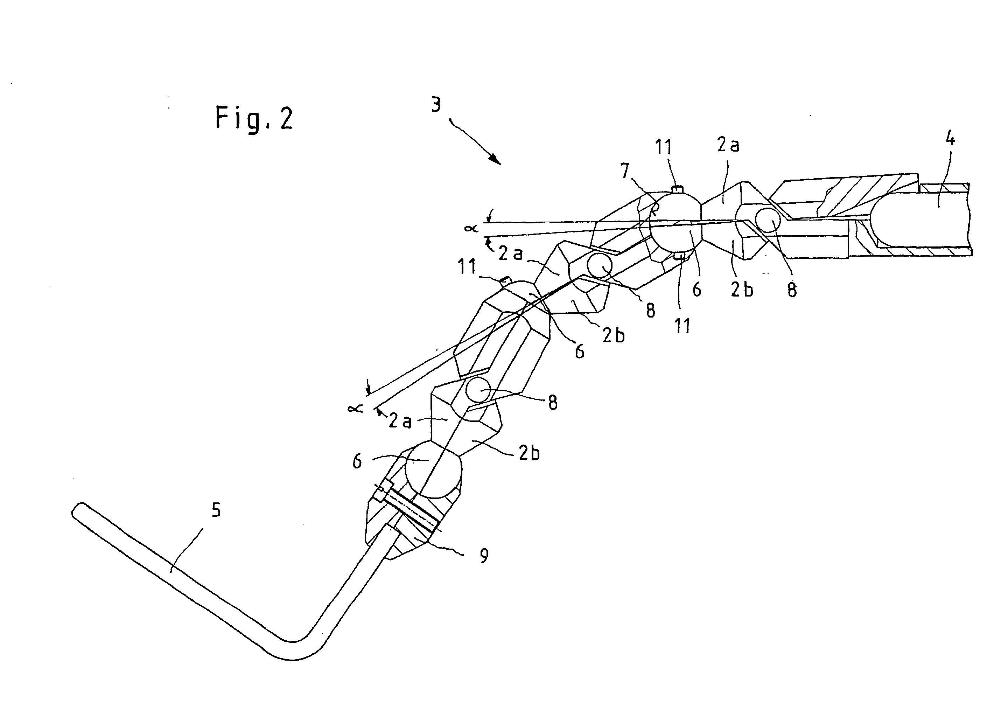

Im Übergangsbereich zwischen Gelenkkugelteil 6 und Gelenkpfannenteil 7 sind die beiden Längsteile 2a und 2b über einen in der Ebene des Längsschnitts querverlaufenden Achsbolzen 8 miteinander verbunden. Diese beiden Längsteilen 2a und 2b gemeinsame Achse 8 ermöglicht ein solches Verschwenken der Längsteile 2a, 2b gegeneinander, daß zwischen den Teilen der Gelenkkugel 6 und den Teilen der Gelenkpfanne 7 ein Öffnungswinkel $\alpha$ ausgebildet wird, wie dies insbesondere der vergrößerten Darstellung gemäß Fig. 2 zu entnehmen ist.

Durch das Spreizen der Gelenkpfanne 7 eines Gliederelements 2 wird über die Achse 8 gleichzeitig die Gelenkkugel 6 desselben Gliederelements 2 gespreizt, wodurch diese sich in der Gelenkpfanne 7 des nachfolgenden Gliederelements 2 verklemmt und diese Gelenkpfanne 7 aufspreizt. Dieses gegenseitige Aufspreizen von Gelenkpfanne 7 und Gelenkkugel 6 führt zu einer vollständigen Versteifung des flexiblen Arms 3, so daß dieser in der jeweiligen Lage fixiert ist. Wie insbesondere aus Fig. 2 ersichtlich, nimmt der Öffnungswinkel $\alpha$ vom proximalen Ende des Arms 3 hin zum distalen Ende von Gliederelement 2 zu Gliederelement 2 wie folgt ab

$$\alpha_{prox} > \alpha_n > \alpha_{n+1} > \cdots > \alpha_{dist} \cong 0.$$

Bei dem dargestellten Ausführungsbeispiel einer Spannvorrichtung 1 erfolgt das Aufspreizen der Gelenkpfanne 7 des proximalen Gliederelements 2 über die als Spannelement dienende Handhabe 4. Das am distalen Ende des Arms 3 angeordnete Werkzeug 5 ist über ein Verbindungselement 9 mit der Gelenkkugel 6 des distalen Gliederelements 2 verbunden.

Um einerseits die Gliederelemente 2 beim gegenseitigen Verschwenken zu führen und andererseits den Verschwenkwinkel zu begrenzen, weist jede Gelenkkugel 6 rechtwinklig zur Längsteilung eine Durchgangsbohrung 10 im Bereich des Kugeläquators auf. In die Durchgangsbohrungen 10 jedes Gelenkkugelteils 6 wird ein Stift 11 so eingesetzt, daß dieser über die Kugeloberfläche der Gelenkkugel 6 herausragt. In jeder Gelenkpfanne 7 ist eine entsprechende Nut 12 zur Aufnahme der Stifte 11 ausgebildet. Die inneren Ränder der Nut 12 dienen dabei als Anschlagfläche 13 zur Begrenzung des Verschwenkwinkels.

Eine solchermaßen ausgebildete Spannvorrichtung 1 zeichnet sich dadurch aus, daß der Arm 3 in nahezu beliebeige Richtungen verstellt und in der jeweiligen Lage arretiert werden kann. Der einfache Aufbau der einzelnen Gliederelemente 2 ohne versteckte Hohlräume ermöglicht darüber hinaus ein leichtes und vollständiges Reinigen der Spannvorrichtung 1.

Neben der dargestellten Aufspreizung von Gelenkkugel 6 und Gelenkpfanne 7 ist es auch möglich, das Verklemmen der Gliederelemente 2 dadurch zu erzielen, daß die einzelnen Längsteile 2a und 2b der Gliederelemente 2 gegeneinander verschoben werden. Durch die Aufteilung der Gliederelemente 2 in mindestens zwei gegeneinander verstellbare Längsteile 2a, 2b bewirkt jede Verformung der Gelenkkugel 6 ein Verklemmen in der entsprechenden Gelenkpfanne 7 und somit ein Arretieren des flexiblen Arms 3.

## Bezugszeichenliste

1    Spannvorrichtung

2    Gliederelement

2a   Längsteil

2b   Längsteil

3    Arm

4    Handhabe

5    Werkzeug

6    Gelenkkugel

7    Gelenkpfanne

8    Achse / Achsbolzen

9    Verbindungselement

10   Durchgangsbohrung

11   Stift

12   Nut

13   Anschlagfläche

α    Öffnungswinkel

## Patentansprüche

1. Flexible Spannvorrichtung, insbesondere für medizinische Zwecke, mit einem aus mehreren gegeneinander verschwenkbaren Gliederelementen (2) bestehenden Arm (3), wobei der Arm (3) in der jeweiligen Verschwenkstellung der Gliederelemente (2) über ein Spannelement arretierbar ist, und wobei jedes Gliederelement (2) an einem Ende eine Gelenkkugel (6) und an dem gegenüberliegenden Ende eine Gelenkpfanne (7) zur Aufnahme einer Gelenkkugel (6) aufweist, **dadurch gekennzeichnet, daß** jedes Gliederelement (2) in Längsrichtung zweiteilig aufgebaut ist, wobei die einzelnen Längsteile (2a, 2b) zum Arretieren gegeneinander verstellbar sind.

2. Spannvorrichtung nach Anspruch 1, **dadurch gekennzeichnet, daß** die Längsteile (2a, 2b) der in Längsrichtung zweiteilig aufgebauten Gliederelemente (2) in der Seitenansicht in etwa Z-förmig so ausgebildet sind, daß bei einem der Längsteile (2a) der obere Teil der Gelenkkugel (6) mit dem unteren Teil der Gelenkpfanne (7) und bei dem anderen Längsteil (2b) desselben Gliederelements (2) der untere Teil der Gelenkkugel (6) mit dem oberen Teil der Gelenkpfanne (7) verbunden ist.

3. Spannvorrichtung nach Anspruch 1 oder 2, **dadurch gekennzeichnet, daß** die Längsteile (2a, 2b) eines jeden Gliederelements (2) über eine gemeinsame Achse (8) gegeneinander verschwenkbar sind.

4. Spannvorrichtung nach Anspruch 3, **dadurch gekennzeichnet, daß** die Achse (8) zum Verschwenken der Längsteile (2a, 2b) eines jeden Gliederelements (2) in der Mitte des Gliederelements (2) angeordnet und in der Ebene des Längsschnitts durch Gelenkkugel (6) und Gelenkpfanne (7) so ausgebildet ist, daß die Gelenkkugel (6) und die Gelenkpfanne (7) einen Öffnungswinkel (α) zwischen den Längsteilen (2a, 2b) ausbildend spreizbar sind.

5. Spannvorrichtung nach Anspruch 4, **dadurch gekennzeichnet, daß** die Öffnungswinkel (α) ausgehend vom proximalen Gliederelement (2) hin zum distalen Gliederelement (2) von Gliederelement (2) zu Gliederelement (2) mit

$$\alpha_{prox} > \alpha_n > \alpha_{n+1} > \cdots > \alpha_{dist} \cong 0$$

abnehmen.

**6.** Spannvorrichtung nach mindestens einem der Ansprüche 1 bis 5, **dadurch gekennzeichnet, daß** rechtwinklig zur Längsteilung der Gelenkkugel (6) durch den Kugeläquator der Gelenkkugel (6) eines jeden Gliederelements (2) eine Durchgangsbohrung (10) ausgebildet ist, in die pro Längsteil (2a, 2b) der Gelenkkugel (6) ein nach außen über die Oberfläche der Gelenkkugel (6) herausragender Stift (11) einsetzbar ist, der zum Führen und Begrenzen des Verschwenkwinkels der einzelnen Gliederelemente (2) gegeneinander im mit einem anderen Gliederelement (2) verbundenen Zustand in einer in der Gelenkpfanne (7) ausgebildeten Nut (12) geführt wird.

**7.** Spannvorrichtung nach mindestens einem der Ansprüche 1 bis 6, **dadurch gekennzeichnet, daß** an das das proximale Ende des Arms (3) bildende Gliederelement (2) eine ein Spannelement bildened Handhabe (4) zum Verstellen und Arretieren der Gliederelemente (2) anschließbar ist.

**8.** Spannvorrichtung nach mindestens einem der Ansprüche 1 bis 7, **dadurch gekennzeichnet, daß** am das distale Ende des Arms (3) bildende Gliederelement (2) ein Werkzeug (5) festlegbar ist.

## Claims

**1.** Flexible tensioning device, especially for medical purposes, with an arm (3) consisting of a plurality of link elements (2) which can be pivoted relative to one another, said arm (3) being lockable via a tensioning element in the respective pivot position of the link elements (2), each link element (2) having a ball (6) at one end and a socket (7) at the other end for receiving a ball (6), **characterized in that** each link element (2) has a two-part construction in the longitudinal direction, the individual longitudinal parts (2a, 2b) being adjustable relative to one another for locking.

**2.** Tensioning device according to Claim 1, **characterized in that** the longitudinal parts (2a, 2b) of the link elements (2) with a two-part construction in the longitudinal direction are of approximately Z-shaped design in side view so that, in one of the longitudinal parts (2a), the upper part of the ball (6) is connected to the lower part of the socket (7) and, in the other longitudinal part (2b) of the same link element (2), the lower part of the ball (6) is connected to the upper part of the socket (7).

**3.** Tensioning device according to Claim 1 or 2, **characterized in that** the longitudinal parts (2a, 2b) of each link element (2) can be pivoted relative to one another via a common axle (8).

**4.** Tensioning device according to Claim 3, **characterized in that** the axle (8) for pivoting the longitudinal parts (2a, 2b) of each link element (2) is arranged at the centre of the link element (2) and, in the plane of the longitudinal section through ball (6) and socket (7), is designed such that the ball (6) and the socket (7) can be spread to form an opening angle ($\alpha$) between the longitudinal parts (2a, 2b).

**5.** Tensioning device according to Claim 4, **characterized in that**, starting from the proximal link element (2) and moving towards the distal link element (2), the opening angle ($\alpha$) decreases from link element (2) to link element (2) by

$$\alpha_{prox} > \alpha_n > \alpha_{n+1} > ... > \alpha_{dist} \cong 0.$$

**6.** Tensioning device according to at least one of Claims 1 to 5, **characterized in that**, at right angles to the longitudinal division of the ball (6), a throughbore (10) is formed through the equator of the ball (6) of each link element (2), into which through-bore (10) a pin (11) can be inserted per longitudinal part (2a, 2b) of the ball (6), which pin (11) protrudes outwards beyond the surface of the ball (6) and, for the purpose of guiding and limiting the pivot angle of the individual link elements (2) relative to one another, is guided in a groove (12) formed in the socket (7), when in the state connected to another link element (2).

**7.** Tensioning device according to at least one of Claims 1 to 6, **characterized in that** a handle (4) forming a tensioning element for adjusting and locking the link elements (2) can be connected to the link element (2) forming the proximal end of the arm (3).

**8.** Tensioning device according to at least one of Claims 1 to 7, **characterized in that** a tool (5) can be secured on the link element (2) forming the distal end of the arm (3).

## Revendications

**1.** Dispositif de serrage souple, en particulier pour des usages médicaux, comportant un bras (3) constitué par plusieurs éléments-maillons (2) mobiles en basculement les uns par rapport aux autres, le bras (3) pouvant être bloqué dans la position de basculement respective des éléments-maillons (2) via un élément de serrage, et chaque élément-maillon (2) comprenant à une extrémité une rotule d'articulation (6) et à l'extrémité opposée une cavité d'articulation (7) pour recevoir une rotule d'articulation (6), **caractérisé en ce que** chaque élément-maillon (2) est conçu en deux pièces en direction longitudinale, les pièces longitudinales individuelles (2a, 2b) étant déplaçables l'une par rapport à l'autre pour le blocage.

2.  Dispositif de serrage selon la revendication 1, **caractérisé en ce que** les pièces longitudinales (2a, 2b) des éléments-maillons (2) conçus en deux pièces en direction longitudinale sont réalisées, en vue de côté, approximativement en forme de Z, de telle sorte que pour l'une des pièces longitudinales (2a) la partie supérieure de la rotule (6) est reliée à la partie inférieure de la cavité (7) et pour l'autre pièce longitudinale (2b) du même élément-maillon (2) la partie inférieure de la rotule (6) est reliée à la partie supérieure de la cavité (7).

3.  Dispositif de serrage selon l'une ou l'autre des revendications 1 et 2, **caractérisé en ce que** les pièces longitudinales (2a, 2b) de chaque élément-maillon (2) sont mobiles en basculement l'une par rapport à l'autre via un axe commun (8).

4.  Dispositif de serrage selon la revendication 3, **caractérisé en ce que** l'axe (8) pour le basculement des pièces longitudinales (2a, 2b) de chaque élément-maillon (2) est agencé au milieu de l'élément-maillon (2) et est réalisé dans le plan de la coupe longitudinale à travers la rotule (6) et la cavité (7), de telle sorte que la rotule (6) et la cavité (7) sont écartables en formant un angle d'ouverture ($\alpha$) entre les pièces longitudinales (2a, 2b).

5.  Dispositif de serrage selon la revendication 4, **caractérisé en ce qu'**à partir de l'élément-maillon proximal (2) vers l'élément-maillon distal (2), les angles d'ouverture ($\alpha$) diminuent d'un élément-maillon (2) à l'autre, suivant la relation

$$\alpha\text{prox} > \alpha n > \alpha n+1 > ... > \alpha\text{dist} \cong 0.$$

6.  Dispositif de serrage selon l'une des revendications 1 à 5, **caractérisé en ce qu'**un perçage traversant (10) est ménagé à angle droit par rapport à la division longitudinale de la rotule (6) à travers l'équateur de la rotule (6) de chaque élément-maillon (2), perçage dans lequel peut être mise en place, pour chaque pièce longitudinale (2a, 2b) de la rotule (6), une tige (11) en saillie vers l'extérieur au-delà de la surface de la rotule (6), tige qui est guidée dans une gorge (12) ménagée dans la cavité (7) pour guider et limiter l'angle de basculement des éléments-maillons individuels (2) les uns par rapport aux autres, dans l'état relié à un autre élément-maillon (2).

7.  Dispositif de serrage selon l'une des revendications 1 à 6, **caractérisé en ce qu'**à l'élément-maillon (2) formant l'extrémité proximale du bras (3), peut être raccordée une manette (4) qui forme un élément de serrage et qui est destinée à déplacer et bloquer les éléments-maillons (2).

8.  Dispositif de serrage selon l'une des revendications 1 à 7, **caractérisé en ce qu'**un outil (5) peut être immobilisé sur l'élément-maillon (2) formant l'extrémité distale du bras (3).

Fig.1

Fig. 2

Fig. 3a

Fig. 3b